# EUROPÄISCHE PATENTSCHRIFT

(11) **EP 1 131 005 B1**
(45) Veröffentlichungstag und Bekanntmachung des Hinweises auf die Patenterteilung: **19.05.2004**
(21) Anmeldenummer: 98952497.0
(22) Anmeldetag: 20.11.1998
(51) Int. Cl.: A61B 17/02

(54) **RETRAKTIONSPLATTE FÜR WEICHTEILE**
RETRACTION PLATE FOR SOFT-TISSUE PARTS
PLAQUE DE RETRACTION POUR PARTIES MOLLES

(43) Veröffentlichungstag der Anmeldung: 12.09.2001
(73) Patentinhaber: SYNTHES AG Chur, 7002 Chur (CH)
(72) Erfinder: RUPP, Stephan, CH-7260 Davos-Dorf (CH); HEHLI, Markus, CH-7276 Frauenkirch (CH)
(74) Vertreter: Lusuardi, Werther Giovanni, Dr.
(86) Internationale Anmeldenummer: PCT/CH1998/000499
(87) Internationale Veröffentlichungsnummer: WO 2000/030549

(56) Entgegenhaltungen:
- WO-A-98/48704
- DE-A- 4 343 117
- DE-U- 29 507 637
- US-A- 5 520 608
- US-A- 5 681 311
- US-A- 5 716 326
- US-A- 5 803 903

## Beschreibung

Die Erfindung betrifft eine Retraktionsplatte gemäss dem Oberbegriff des Patentanspruchs 1 und einen Retraktionsapparat gemäss dem Oberbegriff des Patentanspruchs 17.

Der aus einer Retraktionsplatte und zwei damit lösbar verbindbaren stabförmigen Elementen bestehende Retraktionsapparat dient dazu bei chirurgischen Verfahren die Weichteile temporär zu verschieben um eine Kavität in den Extremitäten oder im Rumpf zu erzeugen. Eine Retraktionsplatte gemäss dem Oberbegriff des Anspruchs 1 ist bereits aus der WO98/48704 bekannt. Nachteilig bei dieser bekannten Platte ist der Umstand, dass sie nur ein einziges Mittel aufweist um die Platte mit einem Manipulationsinstrument lösbar zu verbinden. Die Manipulation der Platte ist deshalb umständlich und unsicher.

Der Erfindung liegt die Aufgabe zugrunde, eine Vorrichtung zu schaffen, mit welcher es möglich ist intraoperativ die Weichteile vom Knochen rasch und sicher abzuheben, um eine Kavität zu schaffen, damit das auszuführende chirurgische Verfahren einfacher und sicherer durchgeführt werden kann.

Die Erfindung löst die gestellte Aufgabe mit einer Retraktionsplatte, welche die Merkmale des Anspruchs 1 aufweist und einem Retraktionsapparat, welcher die Merkmale des Anspruchs 17 aufweist.

Damit ist der Vorteil erzielbar, eine Fraktur mit einer minimal invasiven Technik umsorgen zu können. Die Reposition der Fraktur und die Lage der Retraktionsplatte oder Retraktionsscheibe kann mit einer Optik kontrolliert werden. Anschliessend kann die Verschraubung mit einer minimal invasiven Technik durchgeführt werden. Dies hat die Vorteile, dass durch die Kontrolle der Reposition weniger Fehlstellungen in der ausgeheilten Fraktur auftreten. Auch treten weniger Komplikationen durch falsch plazierte Platten/Fixateure auf. Durch die minimal invasive Technik wird die Durchblutung weniger gestört, welche sehr wichtig für den Heilungsvorgang ist. Eine schnellere Heilung ist möglich, und damit wird die Hospitalisierungszeit verkürzt. Auch kosmetisch hat die mit der erfindungsgemässen Retraktionsplatte oder Retraktionsscheibe durchführbare neue Operationstechnik Vorteile für den Patienten.

Alle nachfolgenden Ausführungen werden anhand der Retraktionsplatte beschrieben, analoges gilt jedoch auch für die Retraktionsscheibe, welche funktionell den beiden Endteilen der Retraktionsplatte entspricht.

Da in den Extremitäten keine natürliche Kavität vorhanden ist, muss diese erst erzeugt werden. Dies kann zum Beispiel mit einem ballonförmigen, aufblasbaren Instrument gemacht werden. Dabei werden zwei Faszien getrennt, zwischen denen anschliessend die Kavität mit dem Plattenretraktor gehalten wird. Nachdem das ballonförmige Instrument entfernt wird, kann der Plattenretraktor durch die Hauptinzision, welche an geeigneter Stelle angebracht wird, zwischen die getrennten Faszien geschoben werden. Die Retraktorenplatte kann anschliessend mit lösbar befestigten stabförmigen Elementen hochgezogen werden, so dass zwischen Retraktionsplatte und Knochen die gewünschte Kavität entsteht.

Um die Retraktionsplatte besser manipulieren zu können empfiehlt sich die Verwendung einer Zange mit Arretierung, welche in einer geeigneten korrespondierenden Vertiefung der Retraktionsplatte eingreifen kann, so dass damit eine bessere Führung erreicht wird, d.h. die Retraktionsplatte kann sich nicht mehr verdrehen.

Die Retraktionsplatte kann in verschiedenen Längen und Formen hergestellt werden. Durch manuelles oder instrumentelles Anbiegen kann die Retraktionsplatte an die aktuelle operative Situation angepasst werden.

Die Retraktionsplatte weist in ihrem Mittelteil eine Durchgangsöffnung auf, um ein Arbeiten von oben durch dieses "Fenster" zu ermöglichen. Durch dieses "Fenster" kann beispielsweise eine Osteosyntheseplatte oder ein Fixateur verschraubt werden. Für diesen Fall kann der "Fensterrahmen" im Mittelteil der Retraktionsplatte auf zwei relativ dünne Träger reduziert werden, welche damit leicht angebogen werden können, um sie der jeweiligen Situation anzupassen.

Nach erfolgter Einführung der Retraktionsplatte unter die Weichteile, werden die dazugehörigen stabförmigen Elemente oder Dorne, an denen die Retraktionsplatte aufgehängt werden soll, von oben durch die Weichteile hindurchgestochen. Die stabförmigen Elemente greifen dann mit ihren am freien Ende angebrachten Verbindungsmitteln in die in den Endteilen der Retraktionsplatte untergebrachten korrespondieren Mittel ein, so dass eine lösbare Verbindung mit den stabförmigen Elementen zustande kommt. Um eine blind erfolgende Suche (allenfalls auch mit einer Optik unterstützbar) des korrespondierenden Mittels in den Endbereichen der Retraktionsplatte zu erleichtern, sind eine Mehrzahl davon auf der Retraktionsplatte angeordnet. Es genügt dann, dass das stabförmige Element auf eines dieser Mittel stösst und temporär verriegelt werden kann.

Um die temporäre Verriegelung zwischen Retraktionsplatte und je einem rechts und links in die Endteile eingeführten stabförmigen Element zu erreichen, sind verschiedenartige Systeme möglich.

Ein erstes besteht darin, die Mittel in den Endteilen als kreiszylindrische Bohrungen mit Innengewinde auszugestalten. Die stabförmigen Elemente tragen dann an ihrem freien Ende ein korrespondierendes Aussengewinde. Die korrespondierenden Gewinde können bei einer zweiten Ausführungsform auch konisch ausgebildet werden, so dass das stabförmige Element lediglich eine halbe oder ganze Umdrehung eingedreht werden muss, statt mehrere Umdrehungen, wie dies bei einem kreiszylindrischem Gewinde notwendig ist. Bei einer dritten Ausführungsform bestehen die korrespondierenden Verbindungsmittel aus einem Langloch und einer quer dazu stehenden Vertiefung. Das stabförmige Element hat an seinem unteren Ende einen entsprechenden Querbalken, welcher durch das Langloch eines der Mittel des Endteils hindurchgestossen wird. Anschliessend wird das stabförmige Element 90° um seine Längsachse rotiert und zurückgezogen. Jetzt ist das stabförmige Element in die senkrecht zu dem durchlaufenden Langloch stehende Vertiefung eingerastet. In dieser eingerasteten Position kann sich das stabförmige Element immer noch in einem Kegel von einigen Graden bewegen.

Die mit der Retraktionsplatte temporär verbundenen stabförmigen Elemente können nun an einer externen Aufhängung aufgehängt werden, um die Weichteile ein Stück weit abzuheben.

Die durch die Erfindung erreichten Vorteile sind im wesentlichen darin zu sehen, dass dank der erfindungsgemässen Retraktionsplatte eine künstliche Kavität zwischen Weichteilmantel und Knochen erzeugt werden kann, welche einen freien Blick auf die Fraktur und deren Versorgung mit einer Platte/Fixateur in einer minimal invasiver Technik ermöglicht.

Die Anwendung der Retraktionsplatte zusammen mit den stabförmigen Elementen erlaubt ein ungestörtes Arbeiten durch die Hauptinzision. Die Retraktionsplatte versperrt nicht den Zugang zur Kavität durch irgendwelche Aufbauten. Auch ist es möglich, eine Optik zur Kontrolle der Arbeitsschritte einzubringen. Das Fenster der Retraktionsplatte ermöglicht eine Verschraubung/Verplattung der Fraktur von oben durch eine oder mehrere zusätzliche kleine Stichinzisionen in einer minimal invasiven Technik. Der Anteil des stabförmigen Elementes, welcher aus dem Körper herausragt, kann an einer Haltevorrichtung fixiert werden, um das auszuführende chirurgische Verfahren durchführen zu können.

Die Erfindung und Weiterbildung der Erfindung werden im folgenden anhand der teilweise schematischen Darstellungen mehrere Ausführungsbeispiele noch näher erläutert.

Es zeigen:
Fig. 1 eine Aufsicht auf die erfindungsgemässe Retraktionsplatte;
Fig. 2 eine Seitenansicht der erfindungsgemässe Retraktionsplatte;
Fig. 3 einen Detailquerschnitt durch ein Mittel zur lösbaren Verbindung mit senkrecht zur Platte stehenden, stabförmigen Elementen;
Fig. 4 einen Detailquerschnitt durch ein alternatives Mittel zur lösbaren Verbindung mit senkrecht zur Platte stehenden, stabförmigen Elementen;
Fig. 5 einen Längsschnitt durch ein stabförmiges Element zur lösbaren Verbindung mit einem Mittel gemäss Fig. 3;
Fig. 6 einen Längsschnitt durch ein stabförmiges Element zur lösbaren Verbindung mit einem Mittel gemäss Fig. 4; und
Fig. 7 einen Längsschnitt durch ein stabförmiges Element zur lösbaren Verbindung mit einem Mittel der Retraktionsplatte gemäss den Fig. 8 bis 10;
Fig. 8 einen Schnitt durch die Retraktionsplatte der Fig. 1 längs der Linie VIII-VIII im Bereich eines Mittels zur lösbaren Verbindung mit senkrecht dazu stehenden, stabförmigen Elementen;
Fig. 9 einen gegenüber der Fig. 8 um 90° verschobenen Schnitt längs der Linie IX-IX in Fig. 1; und
Fig. 10 eine Aufsicht auf ein Mittel zur lösbaren Verbindung mit senkrecht dazu stehenden, stabförmigen Elementen gemäss den Fig. 8 und 9.
Fig. 11 eine perspektivische Ansicht einer scheibenförmigen Retraktionsplatte mit sechs Mitteln zur lösbaren Verbindung mit senkrecht dazu stehenden, stabförmigen Elementen.

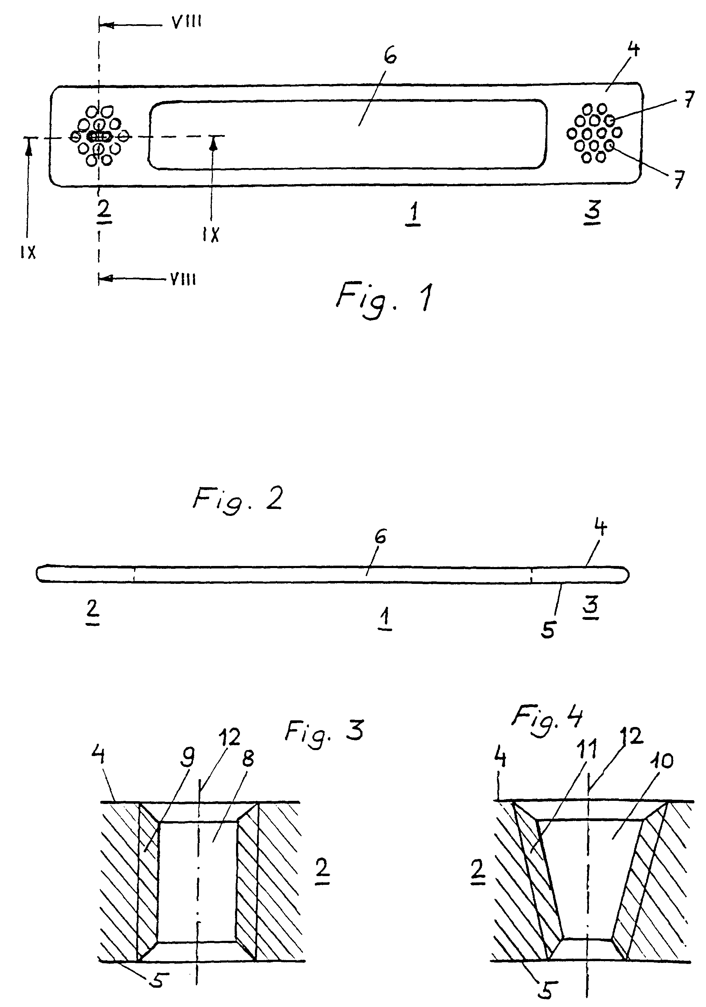

Die in Fig. 1 und 2 dargestellte Retraktionsplatte zum Verschieben von Weichteilen in der Chirurgie umfasst einen Mittelteil 1, zwei durch den Mittelteil 1 voneinander getrennte Endteile 2,3, eine Oberseite 4 und eine Unterseite 5. Der Mittelteil 1 weist eine Durchgangsöffnung 6 in Form eines Fensters auf, welche die Oberseite 4 mit der Unterseite 5 verbindet. Die freie Fläche der Durchgangsöffnung 6 im Mittelteil 1 beträgt 60 % bis 75 %, vorzugsweise 64 % bis 72 % der Oberseite 4 des Mittelteils 1. Bezogen auf die gesamte Oberseite (4) der Retraktionsplatte macht die freie Fläche der Durchgangsöffnung 6 35 % bis 57 %, vorzugsweise 41 % bis 50 % aus. Das Verhältnis F₂₊₃/F₁ der Summe der Flächen F₂₊₃ der Endteile 2,3 zu der Fläche F₁ des Mittelteils 1 liegt im Bereich von 0,40 bis 0,60, vorzugsweise im Bereich von 0,45 bis 0,55.

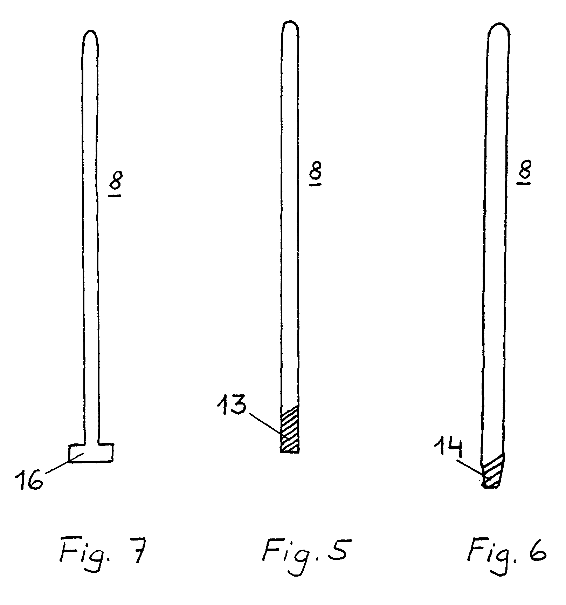

Wie in den Fig. 3 und 4 im Detail dargestellt weisen die Endteile 2,3 der Retraktionsplatte im Bereich ihrer Oberseite 4 eine Mehrzahl von symmetrisch angeordneten Mitteln 7 auf, welche eine lösbare Verbindung mit einem der freien Enden eines stabförmigen Elementes 8 (Fig. 5 bis 7) zulässt.

Bei dem im Fig. 3 dargestellten Mittel 7 handelt es sich um eine kreiszylinderförmige Bohrung 8 mit einem Innengewinde 9, dessen Mittelachse 12 senkrecht zur Retraktionsplatte steht. Ein stabförmiges Element 8 (Fig. 5) mit einem Aussengewinde 13 an einem seiner freien Enden kann in die Bohrung 8 eingeschraubt werden.

Bei dem im Fig. 4 dargestellten Mittel 7 handelt es sich um eine konusförmige Bohrung 10 mit einem Innengewinde 11, wobei sich die konusförmigen Bohrungen 10 von der Oberseite 4 nach der Unterseite 5 hin verjüngt. Die Mittelachse 12 der Bohrung 10 steht dabei senkrecht zur Retraktionsplatte. Ein stabförmiges Element 8 (Fig. 6) mit einem konischen Aussengewinde 14 an einem seiner freien Enden kann in die Bohrung 10 eingeschraubt werden.

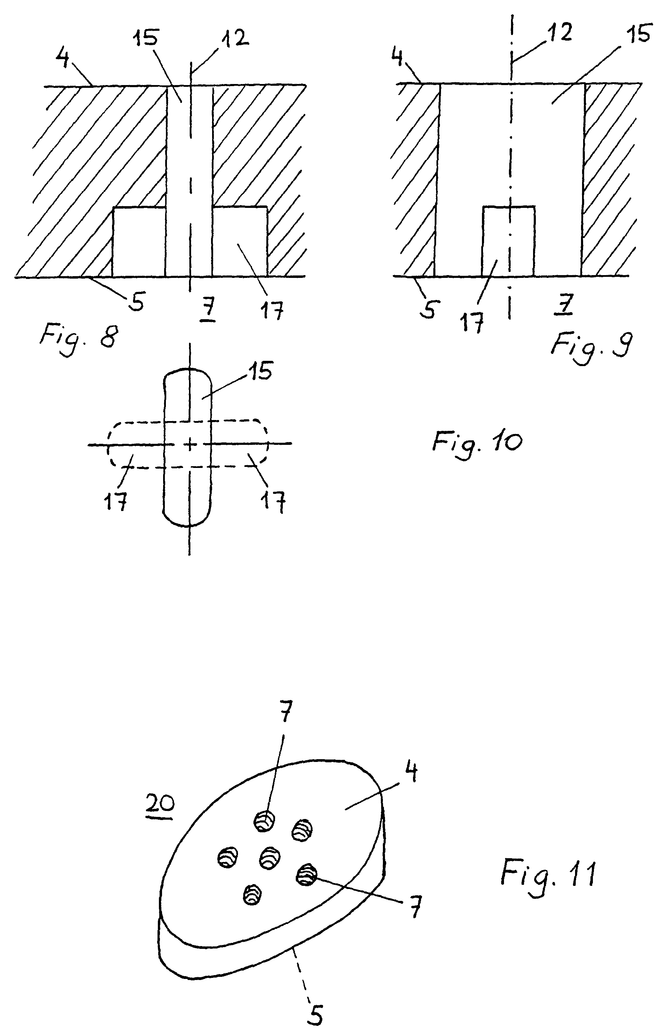

Bei dem in den Fig. 8 - 10 dargestellten Mittel 7 handelt es sich um ein Langloch 15, durch das ein stabförmiges Element 8 (Fig. 7) mit einem Querbalken 16 an seinem unteren Ende geschoben werden kann. Durch Drehen des stabförmigen Elementes 8 (Fig. 7) um 90° können die Querbalken 16 des Elementes 8 in senkrecht zu dem durchgehenden Langloch 15 stehende Vertiefungen 17 eingehängt werden. Dem stabförmigen Element 8 (Fig. 7) ist es im eingehängten Zustand immer noch möglich, sich in einem gewissen Kegel, dessen Achse mit der Mittelachse 12 des Mittels 7 identisch ist, zu bewegen (z.B. innerhalb von 40°).

Statt der in den Figuren 3, 4 sowie 8 - 10 dargestellten Mittel 7 können auch auch andere geeignete Verschlüsse, z.B. Bajonettverschlüsse oder andere flexible Verbindungen verwendet werden.

Die Mittel 7 sind vorzugsweise derart ausgebildet, dass das stabförmige Elemente 8 in vertikaler Richtung zur Retraktionsplatte verbindbar ist; sie können jedoch auch derart ausgebildet werden, dass das stabförmige Element 8 innerhalb eines Konuswinkels, der in einem Bereich von 25° - 50°, vorzugsweise von 35° - 45° liegt, zur vertikalen Richtung der Retraktionsplatte verbindbar ist.

Die in Fig. 11 dargestellte Retraktionsscheibe 20 zum Verschieben von Weichteilen in der Chirurgie weist eine Oberseite 4 und eine Unterseite 5 auf. Die Retraktionsscheibe 20 weist im Bereich ihrer Oberseite 4 eine Mehrzahl von symmetrisch angeordneten Mittel 7 auf, welche eine lösbare Verbindung mit einem der freien Enden eines stabförmigen Elementes 8 (Fig. 5 bis 7) zulässt. Die Retraktionsscheibe 20 entspricht somit funktionell den Endteilen 2,3 der Retraktionsplatte gemäss Fig. 1. Die im Zusammenhang mit der Retraktionsplatte näher ausgeführten speziellen Auführungsformen der Mittel 7 können in analoger Weise auch bei der Retraktionsscheibe 20 angewendet werden.

## Patentansprüche

1. Retraktionsplatte (1;20) zum Verschieben von Weichteilen in der Chirurgie mit einer Oberseite (4) und einer Unterseite (5), **dadurch gekennzeichnet, dass**
im Bereich der Oberseite (4) eine Mehrzahl von Mitteln (7) vorgesehen ist, welche eine lösbare Verbindung mit einem der freien Enden eines stabförmigen Elementes (8) zulässt.

2. Retraktionsplatte nach Anspruch 1, **dadurch gekennzeichnet, dass** sie einen Mittelteil (1) und zwei durch den Mittelteil (1) voneinander getrennte Endteile (2,3) aufweist, wobei der Mittelteil (1) eine Durchgangsöffnung (6) aufweist, welche die Oberseite (4) mit der Unterseite (5) verbindet.

3. Retraktionsplatte nach Anspruch 2, **dadurch gekennzeichnet, dass** die Mehrzahl von Mitteln (7) in den Endteilen (2,3) untergebracht ist.

4. Retraktionsplatte nach Anspruch 2 oder 3, **dadurch gekennzeichnet, dass** die freie Fläche der Durchgangsöffnung (6) im Mittelteil (1) 60 % bis 75 % der Oberseite (4) des Mittelteils (1) ausmacht.

5. Retraktionsplatte nach Anspruch 4, **dadurch gekennzeichnet, dass** die freie Fläche der Durchgangsöffnung (6) im Mittelteil (1) 64 % bis 72 % der Oberseite (4) des Mittelteils (1) ausmacht.

6. Retraktionsplatte nach einem der Ansprüche 2 bis 5, **dadurch gekennzeichnet, dass** die freie Fläche der Durchgangsöffnung (6) im Mittelteil (1) 35 % bis 57 % der gesamten Oberseite (4) der Retraktionsplatte ausmacht.

7. Retraktionsplatte nach Anspruch 6, **dadurch gekennzeichnet, dass** die freie Fläche der Durchgangsöffnung (6) im Mittelteil (1) 41 % bis 50 % der gesamten Oberseite (4) der Retraktionsplatte ausmacht.

8. Retraktionsplatte nach einem der Ansprüche 2 bis 7, **dadurch gekennzeichnet, dass** das Verhältnis F₂₊₃/F₁ der Summe der Flächen F₂₊₃ der Endteile (2,3) zu der Fläche F₁ des Mittelteils (1) im Bereich von 0,40 bis 0,60 liegt.

9. Retraktionsplatte nach Anspruch 8, **dadurch gekennzeichnet, dass** das Verhältnis F₂₊₃/F₁ der Summe der Flächen F₂₊₃ der Endteile (2,3) zu der Fläche F₁ des Mittelteils (1) im Bereich von 0,45 bis 0,55 liegt.

10. Retraktionsplatte nach einem der Ansprüche 1 bis 9, **dadurch gekennzeichnet, dass** die Mittel (7) achsen- und/oder punkt-symmetrisch bezüglich der Retraktionsplatte angeordnet sind.

11. Retraktionsplatte nach einem der Ansprüche 1 bis 10, **dadurch gekennzeichnet, dass** die Mittel (7) aus kreiszylinderförmigen Bohrungen (8) mit einem Innengewinde (9) bestehen, deren Mittelachsen (12) senkrecht zur Retraktionsplatte stehen.

12. Retraktionsplatte nach einem der Ansprüche 1 bis 11, **dadurch gekennzeichnet, dass** die Mittel (7) aus konusförmigen Bohrungen (10) mit einem Innengewinde (11) bestehen, wobei sich die konusförmigen Bohrungen (10) von der Oberseite (4) zur Unterseite (5) hin verjüngen und deren Mittelachsen (12) senkrecht zur Retraktionsplatte stehen.

13. Retraktionsplatte nach einem der Ansprüche 1 bis 12, **dadurch gekennzeichnet, dass** die Mittel (7) aus einem Langloch (15) bestehen, durch das das stabförmige Element (8) mit einem Querbalken (16) an seinem unteren Ende geschoben werden kann.

14. Retraktionsplatte nach einem der Ansprüche 1 bis 13, **dadurch gekennzeichnet, dass** die Mittel (7) aus einem Bajonettverschluss oder einer anderen flexiblen Verbindung bestehen.

15. Retraktionsplatte nach einem der Ansprüche 1 bis 14, **dadurch gekennzeichnet, dass** die Mittel (7) derart ausgebildet sind, dass das stabförmige Element (8) in vertikaler Richtung zur Retraktionsplatte verbindbar ist.

16. Retraktionsplatte nach einem der Ansprüche 1 bis 14, **dadurch gekennzeichnet, dass** die Mittel (7) derart ausgebildet sind, dass das stabförmige Elementen (8) innerhalb eines Kegelwinkels zur vertikalen Richtung der Retraktionsplatte verbindbar ist.

17. Retraktionsapparat zum Verschieben von Weichteilen in der Chirurgie mit einer Retraktionsplatte nach einem der Ansprüche 1 bis 16, **dadurch gekennzeichnet, dass** er mindestens zwei stabförmige Elemente (8) umfasst, welche an einem ihrer freien Enden Mittel umfassen, welche mit den Mitteln (7) der Retraktionsplatte korrespondieren, so dass eine lösbare Verbindung zwischen Retraktionsplatte und stabförmigen Elementen (8) herstellbar ist.

18. Retraktionsapparat nach Anspruch 17, **dadurch gekennzeichnet, dass** die Mittel aus einem kreiszylinderförmigen Aussengewinde (13) bestehen.

19. Retraktionsapparat nach Anspruch 17, **dadurch gekennzeichnet, dass** die Mittel aus einem konischen Aussengewinde (14) bestehen, wobei sich der Durchmesser des Aussengewindes gegen das freie Ende hin verjüngt.

20. Retraktionsapparat nach Anspruch 17, **dadurch gekennzeichnet, dass** die Mittel aus einem Langloch (15) bestehen, wobei die Platte auf der unteren Seite (5) eine senkrecht zum Langloch (15) verlaufende Vertiefung (17) aufweist.

21. Retraktionsapparat nach Anspruch 17, **dadurch gekennzeichnet, dass** die Mittel aus einem Bajonettverschluss oder einer anderen flexiblen Verbindung bestehen.

22. Retraktionsapparat nach einem der Ansprüche 17 bis 21, **dadurch gekennzeichnet, dass** die mit der Retraktionsplatte verbundenen stabförmigen Elemente (8) innerhalb eines Kegelwinkels zur vertikalen Richtung der Retraktionsplatte schwenkbar bleiben.

23. Retraktionsapparat nach Anspruch 22, **dadurch gekennzeichnet, dass** der Kegelwinkel in einem Bereich von 25° - 50°, vorzugsweise von 35° - 45° liegt.

## Claims

1. Retraction plate (1, 20) for shifting of soft parts in surgical operations with an upper side (4) and a lower side (5), **characterised in that**
in the area of the upper side (4) a multiplicity of means (7) is provided which allows a detachable connection with one of the free ends of a rod-shaped element (8).

2. Retraction plate according to Claim 1, **characterised in that** it has a central section (1= and two end sections (2, 3) separated from each other by the central section (1), wherein the central section (1) is provided with a passage opening (6) connecting the upper side (4) to the lower side (5).

3. Retraction plate according to Claim 2, **characterised in that** the majority of the means (7) are located in the end sections (2, 3).

4. Retraction plate according to Claim 2 or Claim 3, **characterised in that** free surface of the passage opening (6) in the central section (1) measures 60% - 75% of the upper side (4) of the central section (1).

5. Retraction plate according to Claim 4, **characterised in that** free surface of the passage opening (6) in the central section (1) measures 64% - 72% of the upper side (4) of the central section (1).

6. Retraction plate according to one of the claims 2 - 5, **characterised in that** the free surface of the passage opening (6) in the central section (1) measures 35% - 57% of the entire upper side (4) of the retraction plate.

7. Retraction plate according to Claim 6, **characterised in that** the free surface of the passage opening (6) in the central section (1) measures 41% - 50% of the entire upper side (4) of the retraction plate.

8. Retraction plate according to one of the claims 2 - 7, **characterised in that** the ratio F₂₊₃/F₁ of the total of the surfaces F₂₊₃ of the end sections (2, 3) to the surface F₁ of the central section (1) is within the range of 0.40 to 0.60.

9. Retraction plate according to Claim 8, **characterised in that** the ratio F₂₊₃/F₁ of the total of the surfaces F₂₊₃ of the end sections (2, 3) to the surface F₁ of the central section (1) is within the range of 0.45 to 0.55.

10. Retraction plate according to one of the claims 1 - 9, **characterised in that** the means (7) are arranged with axial or point symmetry in relation to the retraction plate.

11. Retraction plate according to one of the claims 1 - 10, **characterised in that** the means (7) consist of circular cylindrical holes (8) with an internal thread (9), the central axes (12) of which are positioned vertically to the retraction plate.

12. Retraction plate according to one of the claims 1 - 11, **characterised in that** the means (7) consists of conical holes (8) with an internal thread (11), wherein the conical holes (8) taper from the upper side (4) towards the lower side (5) and the central axes (12) of which are positioned vertically to the retraction plate.

13. Retraction plate according to one of the claims 1 - 12, **characterised in that** the means (7) consist of a longitudinal hole (15), through which the rod-shaped element (8) can be shoved with a transverse beam (16) at its lower end.

14. Retraction plate according to one of the claims 1 - 13, **characterised in that** the means (7) consist of a bayonet catch or some other flexible connection.

15. Retraction plate according to one of the claims 1 - 14, **characterised in that** the means (7) is formed in such a way that the rod-shaped element (8) can be connected in a vertical direction to the retraction plate.

16. Retraction plate according to one of the claims 1 - 14, **characterised in that** the means (7) is formed in such a way that the rod-shaped element (8) can be connected within a conical angle to the vertical direction of the retraction plate.

17. Retraction apparatus for moving soft parts in the course of surgery with a retraction plate according to one of the claims 1 - 16, **characterised in that** it comprises at least two rod-shaped elements (8) comprising means at one of their free ends that correspond to the means (7) of the retraction plate so that a detachable connection is formed between the retraction plate and the rod-shaped elements (8).

18. Retraction plate according to Claim 17, **characterised in that** the means consist of an external thread (13) in circular cylindrical form.

19. Retraction plate according to Claim 17, **characterised in that** the means consist of a conical external thread (14), wherein the diameter of the external thread is tapered towards the free end.

20. Retraction plate according to Claim 17, **characterised in that** the means consist of a longitudinal hole (15), wherein a depression (17) running vertically to the longitudinal hole (15) on the lower side (5).

21. Retraction plate according to Claim 17, **characterised in that** the means consist of a bayonet catch or some other flexible connection.

22. Retraction plate according to one of the claims 17 - 21, **characterised in that** the rod-shaped elements (8) connected to the retraction plate remain with swivel movement within a conical angle to the vertical direction of the retraction plate.

23. Retraction plate according to Claim 22, **characterised in that** the conical angle is within a range of 25 ° - 50°, preferably 35° - 45°.

## Revendications

1. Plaque de rétraction (1;20) pour repousser les parties molles dans la chirurgie avec une face supérieure (4) et une face inférieure (5), **caractérisée en ce que**
dans la zone de la partie supérieure (4), plusieurs organes (7) sont prévus qui permettent une liaison détachable avec l'une des extrémités libres d'un élément en forme de tige (8).

2. Plaque de rétraction selon la revendication 1, **caractérisée en ce qu'**elle présente une partie centrale (1) et deux extrémités (2,3) séparées par la partie centrale (1), la partie centrale (1) présentant un trou traversant (6) qui relie la face supérieure (4) à la face inférieure (5).

3. Plaque de rétraction selon la revendication 2, **caractérisée en ce que** les nombreux organes (7) sont disposés dans les extrémités (2,3).

4. Plaque de rétraction selon la revendication 2 ou 3, **caractérisée en ce que** la surface libre du trou traversant (6) dans la partie centrale (1) occupe 60 % à 75 % de la face supérieure (4) de la partie centrale (1).

5. Plaque de rétraction selon la revendication 4, **caractérisée en ce que** la surface libre du trou traversant (6) dans la partie centrale (1) occupe 64 % à 72 % de la face supérieure (4) de la partie centrale (1).

6. Plaque de rétraction selon une des revendications 2 à 5, **caractérisée en ce que** la surface libre du trou traversant (6) dans la partie centrale (1) occupe 35 % à 57 % de l'ensemble de la face supérieure (4) de la plaque de rétraction.

7. Plaque de rétraction selon la revendication 6, **caractérisée en ce que** la surface libre du trou traversant (6) dans la partie centrale (1) occupe 41 % à 50 % de l'ensemble de la face supérieure (4) de la plaque de rétraction.

8. Plaque de rétraction selon une des revendications 2 à 7, **caractérisée en ce que** le rapport F₂₊₃/F₁ entre la somme des surfaces F₂₊₃ des extrémités (2,3) et la surface F₁ de la partie centrale (1) se situe dans une plage de 0,40 à 0,60.

9. Plaque de rétraction selon la revendication 8, **caractérisée en ce que** le rapport F₂₊₃/F₁ entre la somme des surfaces F₂₊₃ des extrémités (2,3) et la surface F₁ de la partie centrale (1) se situe dans une plage de 0,45 à 0,55.

10. Plaque de rétraction selon une des revendications 1 à 9, **caractérisée en ce que** les organes (7) sont disposés symétriquement par rapport à l'axe et/ou à symétrie ponctuelle en ce qui concerne la plaque de rétraction.

11. Plaque de rétraction selon une des revendications 1 à 10, **caractérisée en ce que** les organes (7) sont constitués d'alésages cylindriques circulaires (8) avec un filetage femelle (9) et dont les axes médians (12) sont perpendiculaires à la plaque de rétraction.

12. Plaque de rétraction selon une des revendications 1 à 11, **caractérisée en ce que** les organes (7) sont constitués d'alésages coniques (10) avec un filetage femelle (11), les alésages coniques (10) diminuant de la surface supérieure (4) en direction de la surface inférieure (5) et dont les axes médians (12) sont perpendiculaires à la plaque de rétraction.

13. Plaque de rétraction selon une des revendications 1 à 12, **caractérisée en ce que** les organes (7) sont constitués d'un trou oblong (15) à travers lequel on peut glisser l'élément en forme de tige (8) avec une barrette transversale (16) sur son extrémité inférieure.

14. Plaque de rétraction selon une des revendications 1 à 13, **caractérisée en ce que** les organes (7) sont constitués d'un verrou baïonnette ou d'un autre raccord flexible.

15. Plaque de rétraction selon une des revendications 1 à 14, **caractérisée en ce que** les organes (7) sont conçus de telle sorte que l'élément en forme de tige (8) peut être relié à la plaque de rétraction dans le sens vertical.

16. Plaque de rétraction selon une des revendications 1 à 14, **caractérisée en ce que** les organes (7) sont conçus de telle sorte que l'élément en forme de tige (8) peut être relié dans un angle conique par rapport au sens vertical de la plaque de rétraction.

17. Rétracteur pour repousser les parties molles dans la chirurgie avec une plaque de rétraction selon une des revendications 1 à 16, **caractérisé en ce qu'**il comprend au moins deux éléments en forme de tige (8) comportant sur une de leurs extrémités libres des organes correspondant aux organes (7) de la plaque de rétraction, de sorte que l'on peut établir une liaison détachable entre la plaque de rétraction et les éléments en forme de tige (8).

18. Rétracteur selon la revendication 17, **caractérisé en ce que** les organes sont constitués d'un filetage mâle cylindrique circulaire (13).

19. Rétracteur selon la revendication 17, **caractérisé en ce que** les organes sont constitués d'un filetage mâle conique (14), le diamètre de ce filetage mâle diminuant en direction de l'extrémité libre.

20. Rétracteur selon la revendication 17, **caractérisé en ce que** les organes sont constitués d'un trou oblong (15), la plaque présentant sur la face inférieure (5) un évidement (17) perpendiculaire au trou oblong (15).

21. Rétracteur selon la revendication 17, **caractérisé en ce que** les organes sont constitués d'un verrou baïonnette ou d'un autre raccord flexible.

22. Rétracteur selon une des revendications 17 à 21, **caractérisé en ce que** les éléments en forme de tige (8) reliés à la plaque de rétraction restent orientables dans un angle conique par rapport au sens vertical de la plaque de rétraction.

23. Rétracteur selon la revendication 22, **caractérisé en ce que** l'angle conique se situe dans une plage de 25° à 50°, de préférence de 35° à 45°.
